(19) Europäisches Patentamt
European Patent Office
Office européen des brevets

(11) **EP 4 614 309 A1**

(12) **EUROPEAN PATENT APPLICATION**

(43) Date of publication:
**10.09.2025 Bulletin 2025/37**

(21) Application number: 24183738.4

(22) Date of filing: **21.06.2024**

(51) International Patent Classification (IPC):
*G06F 7/58* (2006.01)

(52) Cooperative Patent Classification (CPC):
**G06F 7/588**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **04.03.2024 KR 20240030450
15.04.2024 KR 20240050017**

(71) Applicant: **KNU-Industry Cooperation Foundation
Chuncheon-si, Gangwon-do 24341 (KR)**

(72) Inventors:
• JEON, HeeJae
**62246 Gwangju (KR)**
• YOON, Inkwon
**24342 Gangwon-do (KR)**

(74) Representative: **Gille Hrabal
Partnerschaftsgesellschaft mbB
Patentanwälte
Brucknerstraße 20
40593 Düsseldorf (DE)**

(54) **SYSTEM FOR GENERATING RANDOM NUMBERS BASED ON BLOOD SAMPLES AND METHOD USING THE SAME**

(57) According to an aspect of the present invention, there is provided a system for generating random numbers based on blood samples including a sample loading module configured to supply a preset blood sample, a light irradiation module configured to irradiate the blood sample provided by the sample loading module with light, the blood sample moved to a pre-designated region of interest (ROI) being irradiated with light, an image generation module configured to acquire an original speckle image by capturing an image of the blood sample located in the region of interest in a preset method using the light irradiation module, and a random number generation module configured to generate random numbers by processing the original speckle image generated from the image generation module in a preset method.

Fig. 1:

**Description**

CROSS REFERENCE TO RELATED APPLICATION

This application claims priority to Korean Patent Application No. filed on , and all the benefits accruing therefrom under 35 U.S.C. §119, the contents of which are incorporated by reference in their entirety.

BACKGROUND

The present disclosure relates to a system for generating random numbers based on blood samples and a method using the same, and more particularly, to a technology related to a random number generation system using a two-pass tuple-output von Neumann (2P-TO-VN) method.

The content described in this background section simply provides background information for the embodiments and does not constitute prior art.

As the use of wired and wireless communications, including the Internet, is rapidly expanding, the importance of communication network security issues is increasing in terms of protecting important national, corporate, and financial secrets and privacy of individuals. A cryptographic system based on mathematical computational complexity, such as RSA, is considered to be fundamentally decipherable once a quantum computer is developed, and thus countermeasures are required.

Compared to the cryptographic system based on computational complexity, a cryptograph method using one time pad (OTP) is known to be the most secure cryptographic method. When a stream cipher is generated using the one time pad, encrypted communication can be performed quickly and securely. A random number generator (RNG) is used to generate this one time pad.

The evolution and continued development of random number generator systems is laying an important cornerstone for the future of cryptography. This RNG system forms the backbone of all encryption systems due to its role in generating keys that are not only difficult to predict, but also nearly impossible to predict.

Such unpredictability can ensure a high level of security, which is a necessary feature in today's digital world. There are mainly two types of random number generators of algorithmic random number generators and physical random number generators. Algorithmic random number generators also known as pseudorandom number generators (PRNG) use complex mathematical formulas to generate a series of numbers.

The PRNG is convenient and efficient, but it has the disadvantage that the generated random numbers can be reverse engineered and predicted even if they pass a randomness test. On the other hand, the physical (or real) random number generators can derive randomness from stochastic physical processes. Although these processes are theoretically predictable with complete information, they are practically unpredictable due to limitations in time and computational resources.

Due to these characteristics, RNGs play an important role in a variety of cybersecurity tasks, including key generation, digital signature generation, initialization vectors for encryption, and salt value generation for secure storage, and can address the cybersecurity requirements for interconnected systems beyond the IoT domain.

One emerging area of interest is the use of optical and physical unclonable function to enhance security (Di Falco et al). It is illustrated that a chaotic system facilitated by a silicon chip can be exploited to achieve an encryption system having perfect secrecy.

However, it is important to note that physics-based RNG has its own unique challenges. For example, capturing random patterns in a lava lamp is an innovative method, but bandwidth is limited. Another method of using light-emitting diodes and a cell phone camera, captures the randomness in quantum fluctuations of light, but the setup is incredibly complicated. Therefore, the environment of RNG systems has been changing in recent years, and existing artificially created RNGs have been found to contain unique security vulnerabilities.

Because of these vulnerabilities, the transition to a more unpredictable and inherently random bio-inspired RNG systems has been triggered. Changes acknowledge that a biological system often contains a level of complexity and randomness that, if not impossible, are difficult to artificially replicate.

Therefore, recently, the RNG technology is attempting to focus on generating RNGs using speckle patterns. The main goal of these technologies is to achieve a balance between affordability and portability. Both are considered essential for widespread adoption and application of these systems.

Advances in RNGs and physical de-cloning function of photons in the area of speckle pattern generation often rely on the inherent randomness of materials, such as the natural texture of paper, as speckle patterns are generated under coherent light. For example, optical waveguides demonstrate the ability to generate random numbers at Mbit/s with verified randomness.

In addition, an all-optical physical de-cloning function (Fratalocchi et al.) was developed based on the speckle pattern of aerogel to achieve secure key generation. However, these technologies involve passing a laser beam through a volumetric scattering medium to capture the static speckle pattern. Although this approach is innovative, it may pose new challenges for cost-effectiveness and high-speed random number generation. Furthermore, despite the improved security functions provided by these RNGs, their application in industrial settings is limited. The main obstacle lies in the high costs associated with sample production and acquisition speed. If these costs increase, purchasing speed generally improves.

Conversely, lower costs often result in slower speeds, which creates a trade-off that complicates optimization. This problem requires an RNG system that provides excellent performance while significantly reducing manufacturing costs.

As a prior art in the field of RNG, Korean registered patent No. 10-1925787 (Method and device for securing continuity of random number output signal during von Neumann post-processing) is disclosed. The prior art discloses solving the discontinuity of the random number output signal, and has an effect of improving the speed of the system and economizing to lower the cost by storing random numbers in a memory buffer and outputting spare random numbers through another configuration when all the stored random numbers are exhausted,

However, the prior art only discloses random number output technology using von Neumann post-processing, and does not disclose at all the possibility of applying and combining blood flow or blood samples.

## SUMMARY

The present disclosure provides a system that enables high-speed random number generation while maximizing cost-efficiency and also ensures the reliability of the generated random numbers by improving the randomness of the generated random numbers.

However, aspects of the present invention are not limited to the system described above, and other aspects not mentioned will be apparent to those skilled in the art from the following description of the invention.

In accordance with an exemplary embodiment of the present invention, a system for generating random numbers based on blood samples includes a sample loading module configured to supply a preset blood sample, a light irradiation module configured to irradiate the blood sample provided by the sample loading module with light, the blood sample moved to a pre-designated region of interest (ROI) being irradiated with light, an image generation module configured to acquire an original speckle image by capturing an image of the blood sample located in the region of interest in a preset method using the light irradiation module, and a random number generation module configured to generate random numbers by processing the original speckle image generated from the image generation module in a preset method.

Preferably, the random number generation module may include a binary image generation unit configured to generate a binary image by performing a binarization process of classifying pixel values constituting the original speckle image into bit of 0 or 1.

Preferably, the random number generation module may further include a von Neumann extraction unit configured to generate a first processed image by applying a von Neumann post-processing method to the binary image acquired through the binary image generation unit.

Preferably, the von Neumann extraction unit may be configured to generate a second processed image by further applying a two-pass tuple-output von Neumann (2P-TO-VN) method to the first processed image.

Preferably, the von Neumann extraction unit may be configured to apply a one-pass tuple-output von Neumann (1P-TO-VN) method and process two bits as one set in a preset method, and the preset method may be a method of removing one set if the one set is a duplication of the same bits.

Preferably, the von Neumann extraction unit may be configured to, when a set is composed of different bits based on the remaining bits in a state where one set is the duplication of the same bits and the set is removed, maintain only a first bit or second bit constituting the set among the first bit and second bit.

Preferably, the von Neumann extraction unit may be configured to regroup the removed set into a quad through the 1P-TO-VN method, and the quad is composed of a first set and a second set, and determine whether to maintain the removal of the first and second sets based on whether the first and second sets are the same.

Preferably, the von Neumann extraction unit may be configured to restore the first and second sets when the first and second sets are different from each other.

Preferably, the random number generation module may be configured to further include an image scrambling unit configured to generate a pre-processed image by performing image scrambling on the binary image generated through the binary image generation unit, and the von Neumann extraction unit may be configured to generate the first and second processed images for the pre-processed image generated by the image scrambling unit.

In accordance with another exemplary embodiment of the present invention, a method of generating random numbers based on blood samples includes (a1) supplying a blood samples through a sample loading module, (a2) irradiating the blood sample provided by the sample loading module with light through a light irradiation module, the blood sample moved to a pre-designated region of interest (ROI) being irradiated with light, (a3) acquiring an original speckle image by capturing an image of the blood sample located in the region of interest in a preset method using the light irradiation module, and (a4) generating random numbers by processing the original speckle image generated in the (a3) in a preset method.

Preferably, the (a4) may include (a41) generating a binary image by performing a binarization process of classifying pixel values constituting the original speckle image into bit of 0 or 1, (a42) generating a first processed image by applying a von Neumann post-processing method to the binary image acquired in the (a41), and (a43) generating a second processed image by further applying a 2P-TO-VN scheme to the first processed image generated in the (a42).

Preferably, the method may further include, before the (a42), (a42-0) generating a pre-processed image by performing image scrambling on the binary image generated in the (a41).

## BRIEF DESCRIPTION OF THE DRAWINGS

Exemplary embodiments can be understood in more detail from the following description taken in conjunction with the accompanying drawings, and the following drawings attached to this specification illustrate preferred embodiments of the present invention, and serve to further understand the technical idea of the present invention together with the detailed description of the invention described later. Therefore, the present invention should not be construed as limited to the matters described in such drawings, in which:

FIG. 1 is a block diagram illustrating the overall configuration of the system in accordance with an exemplary embodiment of the present invention;

FIG. 2 is a schematic diagram illustrating a blood sample testing device of the system in accordance with the exemplary embodiment of the present invention;

FIG. 3 is a schematic diagram illustrating a sample loading module in the blood sample testing device of FIG. 2;

FIG. 4 is a conceptual diagram schematically illustrating a step of generating random numbers using the system in accordance with the exemplary embodiment;

FIG. 5 is a block diagram schematically illustrating a processing process in a von Neumann extraction unit of the system in accordance with the exemplary embodiment;

FIG. 6 is an example for describing the process in FIG. 5;

FIG. 7 is a flowchart of a method in accordance with another exemplary embodiment of the present invention;

FIG. 8 is a flowchart dividing step S40 of FIG. 7 in detail;

FIG. 9 is a graph obtained by comparing the speckle decorrelation times of an original speckle image (OSI), a first processed image (CVN method), and a second processed image (2P-TO-VN method);

FIG. 10 is a graph obtained by comparing random number matrix characteristics of the original speckle image (OSI), the first processed image (CVN method), and the second processed image (2P-TO-VN method);

FIG. 11 illustrates results of speckle bit generation rates of the first processed image (CVN method) and the second processed image (2P-TO-VN method) and the respective images;

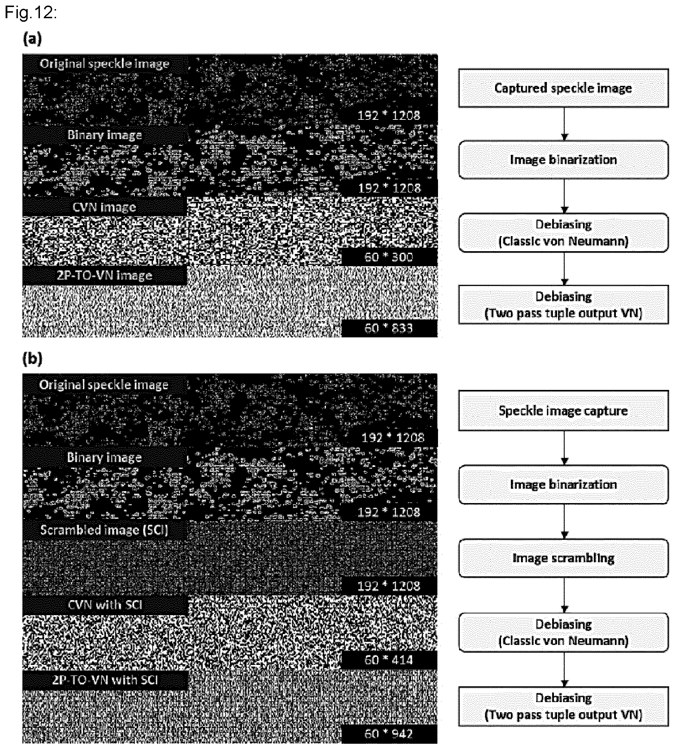

The (a) of FIG. 12 is an exemplary image illustrating the original speckle image (OSI), the first processed image (CVN method), and the second processed image (2P-TO-VN method), and (b) of FIG. 12 illustrates an exemplary image to which image scrambling has been applied; and

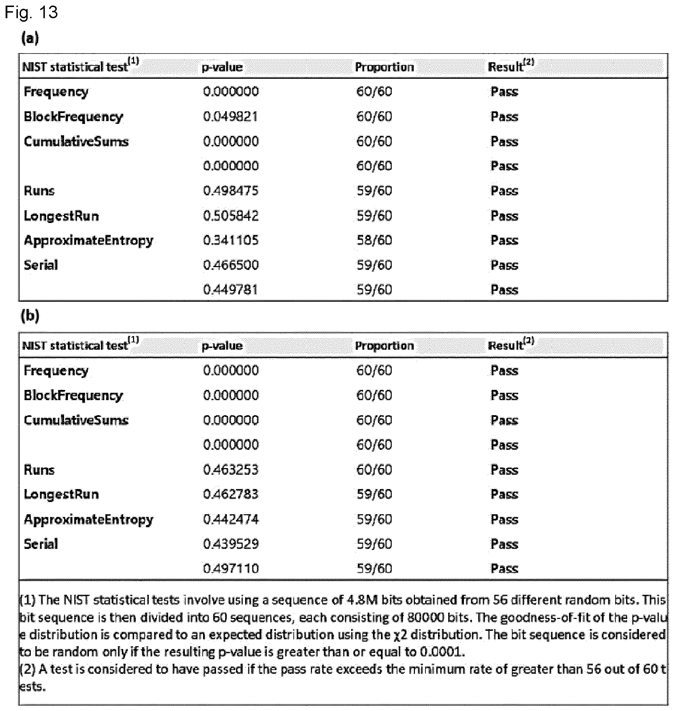

FIG. 13 is a table illustrating the results of the NIST statistical randomness test for random numbers generated using the system in accordance the exemplary embodiment of the present invention.

## DETAILED DESCRIPTION OF EMBODIMENTS

Hereinafter, preferred embodiments of the present invention will be described in detail with reference to the attached drawings. Prior to this, terms and words used in this specification and claims should not be construed as limited to their usual or dictionary meanings, and should be interpreted as meanings and concepts consistent with the technical idea of the present invention based on the principle that the inventor can appropriately define terminology in order to explain his or her invention in the best way.

The present invention may be subjected to various modifications and have several embodiments, and specific embodiments thereof will be illustrated in the drawings and described in detail in the detailed description. However, this is not intended to limit the present invention to specific embodiments, and should be understood to include all modifications, equivalents, and substitutes that fall within s included in the spirit and technical scope of the present invention. In describing each drawing, similar reference numerals are used for similar components.

Terms such as first, second, A, B, etc. can be used to describe various components, but the components should not be limited by the terms. The terms are used only to distinguish one component from another. For example, a first component may be named a second component without departing from the scope of rights of the present invention, and similarly, a second component may also be named a first component. The term "and/or" includes a combination of a plurality of related stated items or any of the plurality of related stated items.

When a component is referred to as being "coupled" or "connected" to another component, it should be understood that the component may be directly coupled or connected to that other component, but other components may exist in between. On the other hand, when a component is referred to as being "directly coupled" or "directly connected" to another component, it should be understood that no other components exist in between.

The terms used in this application are only used to describe specific embodiments and are not intended to limit the present invention. Singular expressions include plural expressions unless the context clearly dictates otherwise. It should be understood that, in this application, terms such as "comprise" or "have" are intended to designate the presence of features, numbers, steps, operations, components, parts, or combinations thereof described in the specification, and do not exclude in advance the possibility of the existence or addition of one or more other features or numbers, steps, operations, components, parts, or combinations thereof.

Unless otherwise defined, all terms used herein, including technical or scientific terms, have the same meaning as generally understood by a person of ordinary skill in the technical field to which the present invention pertains. Terms such as those defined in commonly used dictionaries should be interpreted as having a meaning consistent with the meaning in the context of the related technology, and unless clearly defined in this application, should not be interpreted in an ideal or excessively formal sense.

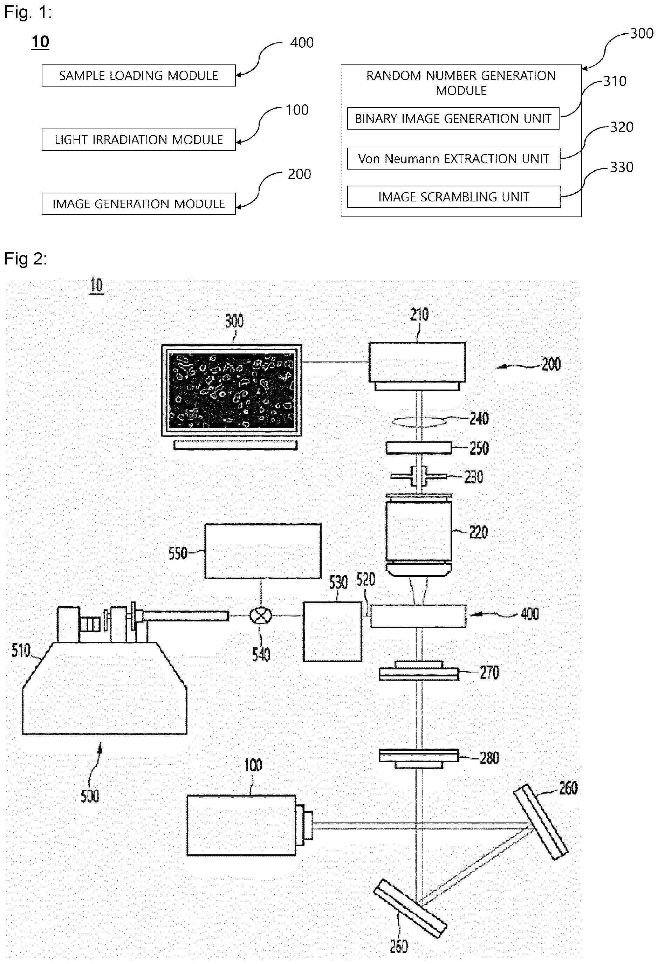

FIG. 1 is a block diagram illustrating the overall configuration of the system according to an embodiment of the present invention.

A system according to an embodiment of the present invention will be described with reference to FIG. 1. A system 10 includes a sample loading module 400, a light irradiation module 100, an image generation module 200, and a random number generation module 300. Here, the random number generation module 300 may be executed as a program stored in a processor included in the system.

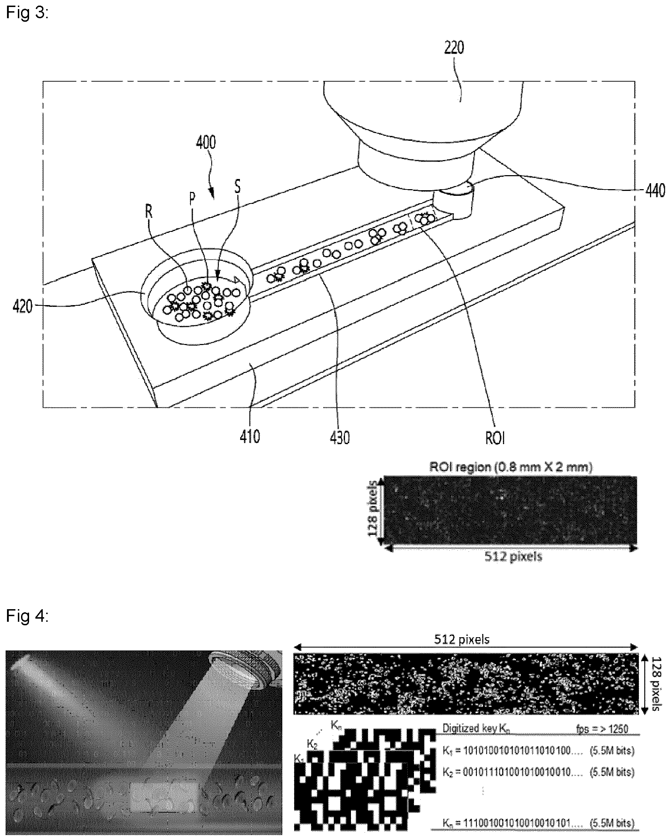

FIG. 2 is a schematic diagram illustrating a blood sample testing device of the system according to embodiment of the present invention, and FIG. 3 is a schematic diagram illustrating a sample loading module in the blood sample testing device of FIG. 2.

Description will be made with reference to FIGS. 2 and 3 together.

The light irradiation module 100 may be configured to irradiate a blood sample S with light. As an example, the blood in the blood sample S may exemplarily include red blood cells R, platelets P, etc., as illustrated in FIG. 3. In addition, the light may be laser light having a wavelength within a certain range (e.g., 532 nm), but is not limited thereto.

The blood sample S may be extracted from a rat and contained in distilled deionized water, but is not limited thereto. The Applicant states that protocol GIST-2019-015 was strictly followed and that all officially approved methods were complied with ARRIVE guidelines for reporting experiments.

Specifically, the Applicant collected blood samples from approximately 12 to 13-week-old male Sprague Dawley rats weighing between approximately 250 and approximately 280 g via tail vein phlebotomy. For this purpose, 1 ml of blood was collected through a 23G needle while the animals were anesthetized with isoflurane. This blood collection was performed on a group of approximately 11 mice, and samples were immediately stored in citrate tubes (Catalog #363083, 9NC 0.109M Buffered Trisodium Citrate, BD Vacutainer, USA) for subsequent experiments.

The image generation module 200 may be configured to acquire a speckle image of the blood sample S irradiated with light. Specifically, the image generation module 200 may include a photographing unit 210, a first lens 220, an aperture 230, a second lens 240, a polarizing plate 250, a reflecting mirror 260, a collimator 270, and a coupler 280.

The photographing unit 210 may be configured to photograph and acquire a speckle image of the blood sample S irradiated with light. As an example, the photographing unit 210 may be a CMOS camera (Neo 5.5 sCMOS, Andor Technology Ltd., Belfast, UK), but is not limited thereto. This CMOS camera operates at a high frame rate of approximately 1250 frames per second with an exposure time of approximately 0.8 milliseconds to smoothly capture speckle images with a resolution of approximately 128x512 pixels for subsequent data generation.

The speckle image of the blood sample S may include a speckle pattern that is irregularly generated by interference that occurs when light is reflected from the blood sample S or light passes through the blood sample S. As an example, a total of approximately 7 experimental data sets were constructed with approximately 1000 frames, and for pre-processing the experimental data, they were divided into approximately 8 partitions, each consisting of approximately 32x256 pixels, for a total of approximately 56 sets were used.

The first lens 220 is an objective lens and may be disposed between the photographing unit 210 and the sample loading module 400 on which the blood sample S is disposed. The aperture 230 is a diaphragm and may be disposed between the photographing unit 210 and the first lens 220. The second lens 240 is a tube lens and may be disposed between the photographing unit 210 and the aperture 230.

The polarizing plate 250 may be disposed between the second lens 240 and the aperture 230.

In this case, the aperture 230, the second lens 240, and the polarizing plate 250 may be provided to improve contrast in the speckle image of the blood sample S acquired by the photographing unit 210.

The reflecting mirror 260 may be configured to reflect light emitted from the light irradiation module 100 and make the light incident on the blood sample S. In this case, at least one reflecting mirror 260 may be provided.

The collimator 270 may be disposed between the sample loading module 400 on which the blood sample S is disposed and the reflecting mirror 260.

The coupler 280 is an optical coupler and may be disposed between the collimator 270 and the reflecting mirror 260.

The sample loading module 400 may be configured to move the blood sample S. As an example, the sample loading module 400 may contain a polydimethylsiloxane (PDMS) material. The sample loading module 400 may include a body 410, an injection unit 420, a movement channel 430, and a discharge unit 440.

The body 410 may configure the overall shape of the sample loading module 400. The injection unit 420 may be a portion through which the blood sample S flows into the body 410.

The movement channel 430 may be provided on the body 410 and may be configured to move the blood sample S introduced through the injection unit 420. Here, the movement channel 430 may be referred to as a 'microchannel'. After the light (e.g., laser light with λ = approximately 532 nm) emitted through the light irradiation module 100 passes through the movement channel 430, an ROI may be captured by the first lens 220. Through this, an 'original speckle image' is acquired, and the original speckle image is constructed to generate random bits by the random number generation module 300, which will be described later.

The movement channel 430 may be designed with precise dimensions of approximately 45 mm in length, approximately 45 μm in height, and approximately 1 mm in width by applying soft photolithography to construct the microchannel. As an example, the Applicant fabricated a PDMS slab according to several standard processes, and the slab composed of PDMS Sylgard 184 A/B (Dow Corning, Korea) was bonded to the cover glass through oxygen plasma treatment.

The movement channel 430 may be composed of a vacuum generator integrated with a pump, a solenoid valve, three-way valve, and a 50ml syringe to account for dead volume. The pump may control sample collection with a constant volume of approximately 200 μL in variable recovery mode using a solenoid valve that regulates the flow.

The discharge unit 440 may be a portion that discharges the blood sample S flowing within the movement channel 430 to the outside of the body 410. A pumping module 500 may include a pump 510, a movement pipe 520, a first valve 530, a second valve 540, and a dead volume chamber 550.

The pumping module 500 may be configured to be connected to the sample loading module 400 and move the blood sample S toward the sample loading module 400 through vacuum pressure.

The pump 510 is a vacuum pump, which may move the blood sample S toward the body 410 of the sample loading module 400 by creating vacuum pressure. As an example, the pump 510 may be a syringe pump. The movement pipe 520 may be a pipe through which the blood sample S is moved toward the body 410 by the vacuum pressure created by the pump 510.

The first valve 530 may be provided in the movement pipe 520 and configured to adjust the vacuum pressure applied to the body 410. As an example, the first valve 530 may be a solenoid valve. The second valve 540 and the dead volume chamber 550 may be provided in the movement pipe 520 to buffer the movement of the blood sample S. In this case, the dead volume chamber 550 may be coupled to the movement pipe 520 through the second valve 540. As an example, the second valve 540 may be a 3-way valve, and the dead volume chamber 550 may be a container formed of an empty space.

The random number generation module 300 is configured to generate random numbers by processing the original speckle image generated from the image generation module 200. As an example, the random number generation module 300 may be implemented in the form of a CPU, GPU, and AP having an operation processing function, or a combination thereof, and may be provided together with DRAM, flash memory, SSD, and various other types of memory as needed.

The random number generation module 300 includes a binary image generation unit 310, a von Neumann extraction unit 320, and an image scrambling unit 330.

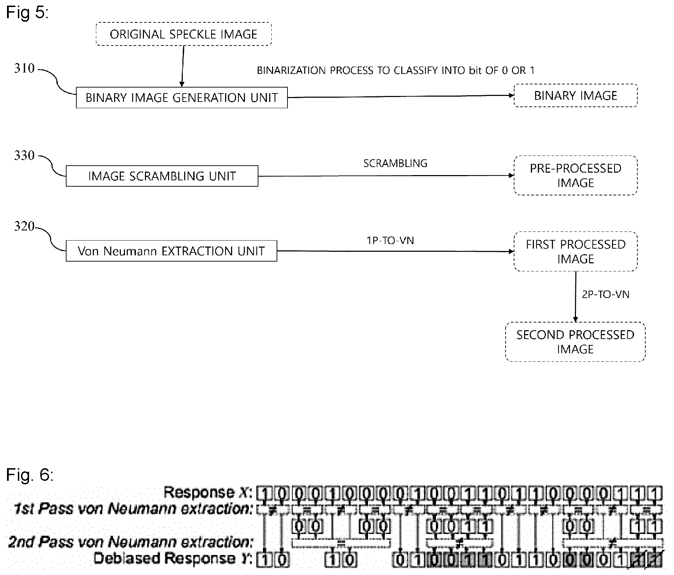

FIG. 4 is a conceptual diagram schematically illustrating a step of generating random numbers using a system according to the embodiment of the present invention and FIG. 5 is a block diagram schematically illustrating a processing process in the von Neumann extraction unit of the system according to the embodiment of the present invention.

The binary image generation unit 310 is configured to generate a binary image by performing a binarization process of classifying pixel values constituting the original speckle image into bit of 0 or 1. That is, the original speckle image is converted into the binary image.

The image scrambling unit 330 is configured to perform scrambling on the binary image. Scrambling code injection (SCI) refers to injecting a scrambling code into the image. The bit generation speed can be improved by scrambling the image before the image is passed through the von Neumann extractor 320.

Both the first and second processed images described later may be images generated with scrambling performed by the image scrambling unit 330.

The von Neumann extractor 320 generates a first processed image by applying the von Neumann post-processing method to the binary image acquired through the binary image generation unit 310. After that, the second processed image can be generated by further applying the two-pass tuple-output von Neumann (2P-TO-VN) method to the first processed image.

Here, the von Neumann post-processing method is in contrast to the two-pass tuple-output von Neumann (2P-TO-VN) method, which may be understood as the one-pass tuple-output von Neumann (1P-TO-VN) method. That is, the 2P-TO-VN method includes a step of going through an additional pass, in addition to the 1P-TO-VN method.

FIG. 6 is an example for describing the process in FIG. 5, and the method of the present invention is not limited to the method illustrated in FIG. 6.

In the von Neumann extraction unit 320, the binary image can be processed in the 2P-TO-VN method by first going through the 1P-TO-VN method and then going through a separate pass again.

Referring to FIG. 6, the binary image goes through a step of separating two bits into one set. Here, if one set is a duplication of the same bits, the set can be removed. As an example, the set of '00' or '11' can be deleted.

Then, in a state where one set is a duplication of the same bits and the set is removed, when a set is composed of different bits based on the remaining bits, the von Neumann extraction unit is configured to maintain only a first bit or second bit constituting the set among the first bit and second bit. In this way, the 1P-TO-VN method is completed. As an example, in the case of '01' or '10', only the first bit, such as '0' of '01' or' 1' of' 10', can be maintained.

After that, the von Neumann extraction unit 320 is configured to perform an additional pass. Through this, the bias of the bit can be lowered.

The 2P-TO-VN method can be understood as regrouping the set removed through the 1P-TO-VN method into a quad, which is composed of a first set and a second set, and further going through a step of determining whether to maintain removal of the first and second sets based on whether the first and second sets are the same. Here, if the first and second sets are different from each other, the first and second sets can be restored. As an example, discarded first and second sets, each of which consists of different bits, within each quad, such as '0011' or '1100', can be set to be saved again.

The classic von Neumann (CVN) debiasing method, which is known as the 1P-TO-VN method, significantly reduces the number of bits due to strict compression, while the 2P-TO-VN method provides an appropriate balance between bit retention and debiasing. The 2P-TO-VN method reevaluates the initially discarded bits to thereby generate a data volume that is more suitable for real applications, as can be seen in FIG. 6, and thus, may be an excellent alternative to the CVN described above for generating strong and usable cryptographic keys (random numbers).

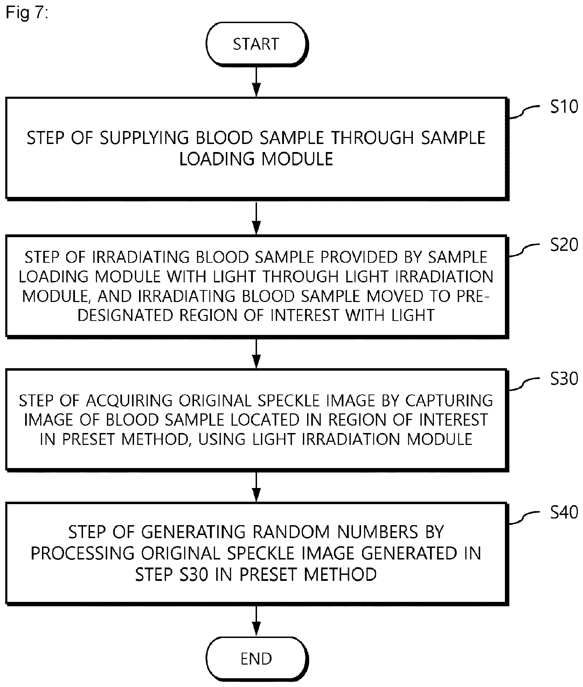

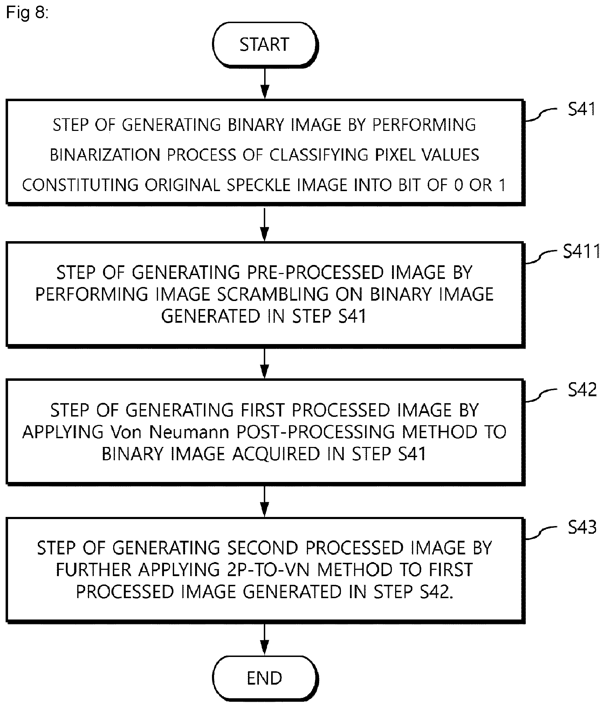

FIG. 7 is a flowchart of a method according to an embodiment of the present invention, and FIG. 8 is a flowchart dividing a step S40 of FIG. 7 in detail.

The method will be described with reference to FIGS. 7 and 8, and descriptions that overlap with the above description will be omitted.

The method according to an embodiment of the present invention includes steps S10 to S40.

Step S10 is a step of supplying a preset blood sample through the sample loading module 400.

Step S20 is a step of irradiating the blood sample provided by the sample loading module 400 with light through the light irradiation module 100, and irradiating the blood sample moved to a pre-designated region of interest with light.

Step S30 is a step of acquiring an original speckle image by capturing an image of the blood sample located in the region of interest in a preset method, using the light irradiation module 100.

Step S40 is a step of generating random numbers by processing the original speckle image generated in the step S30 in a preset method.

Here, step S40 may further include steps S41 to S43.

Step S41 is a step of generating a binary image by performing a binarization process of classifying the pixel values constituting the original speckle image into a bit of 0 or 1.

Step S411 is a step of generating a pre-processed image by performing image scrambling on the binary image generated in the step S41.

Step S42 is a step of generating a first processed image by applying the von Neumann post-processing method to the binary image acquired in the step S41.

Step S43 is a step of generating a second processed image by further applying the 2P-TO-VN method to the first processed image generated in the step S42.

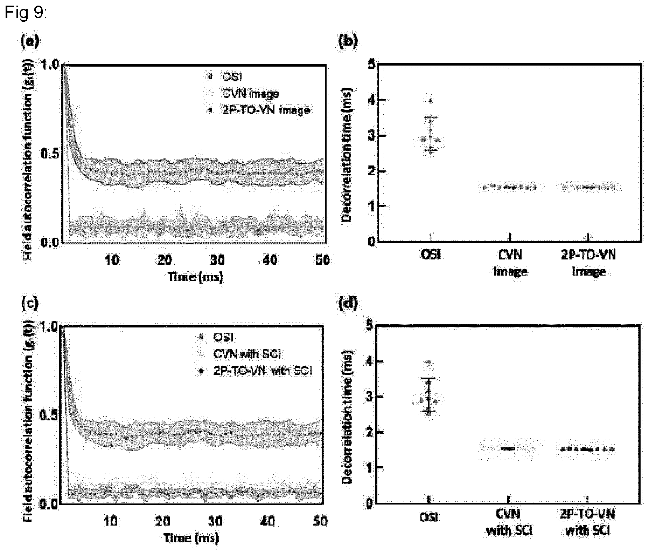

FIG. 9 is a graph obtained by comparing the speckle decorrelation times of the original speckle image (OSI), the first processed image (CVN method), and the second processed image (2P-TO-VN method). The Applicant has performed validation of the results of the method of generating random numbers based on blood flow.

Referring to FIG. 9, the decorrelation time is the main measure of estimated randomness. It shows the autocorrelation curves for approximately 56 original speckle images compared to the real and scrambled random bits. When comparing the original speckle image with the derived random bits, it shows a large difference in the decorrelation time. The average decorrelation time for the original speckle image was approximately 3.05 seconds with a standard deviation of approximately $\pm 0.43$, and a post-processed series of random bits of the classic von Neumann (CVN) image, the CVN with scrambling, the 2P-TO-VN method, and the 2P-TO-VN method with scrambling images show remarkably consistent mean and standard deviation of approximately $1.542 \pm 0.019$, approximately $1.538 \pm 0.018$, approximately $1.551 \pm 0.017$, and approximately $1.529 \pm 0.013$, respectively. These closely clustered values indicate that there is low or no correlation between approximately 56 different images, which means high randomness.

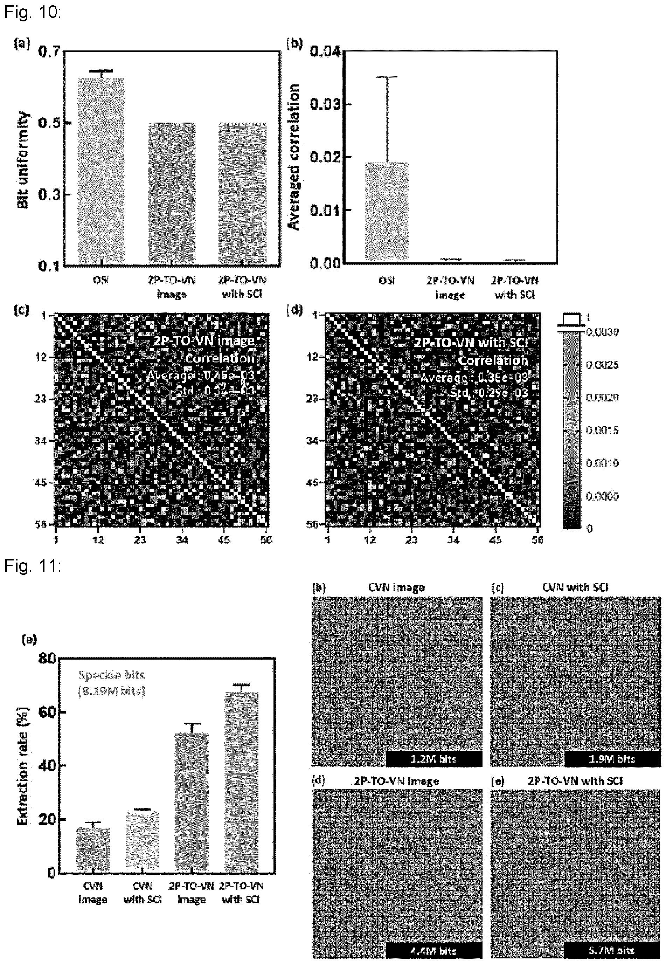

FIG. 10 is a graph obtained by comparing the random number matrix characteristics of the original speckle image (OSI), the first processed image (CVN method), and the second processed image (2P-TO-VN method).

Bit uniformity and correlation difference comparison will be described with reference to FIG. 10. To address potential bias in the data, bit uniformity of the original speckle image with an average of approximately 0.626 was examined by reflecting distortion. After applying the 2P-TO-VN method, an exemplary bit uniformity of approximately 0.500 was checked for both data sets of a case where data is processed only with the 2P-TO-VN method and a case of combined with scrambling code insertion. A numerical value of approximately 0.500 may be understood as illustrating an ideal balance of '1' and '0' bits.

As illustrated in (b) to (d), further investigation using correlation analysis on approximately 56 original images and the processed speckle images shows that the processed dataset achieves a correlation value that is approximately 2,270 times much lower than the original images.

In addition, when comparing the two processed datasets, the 2P-TO-VN method including SCI processing was found to decrease by approximately 18.34% in correlation value compared to the image to which only the 2P-TO-VN method was applied, which can be understood as SCI processing further weakening the correlation.

In (c) and (d), it is shown that the correlation decreases as the correlation value outside the diagonal area approaches approximately 0.

The specific correlation metrics for the original speckle images are found to have a standard deviation of approximately 0.016264 and a mean of approximately 0.01896, and the processed data shows significant improvement in that the 2P-TO-VN images and 2P-TO-VN images with SCI, that respectively have correlation values of approximately 0.000453 and approximately 0.000343, produce much lower correlations of approximately 0.000383 and approximately 0.000296, respectively. These results can strongly emphasize the effectiveness of the 2P-TO-VN method, which achieves bit uniformity and minimizes correlation, and verify the statistical reliability of random number generation based on blood flow speckles.

FIG. 11 illustrates results of speckle bit generation rates of the first processed image (CVN method) and the second processed image (2P-TO-VN method) and the respective images.

Referring to FIG. 11, (a) is a bit generation rate graph generated from the original speckle bits. The speckle bits may consist of approximately 8.1 million bits. The means and standard deviations of the the respective images are calculated to be approximately $16.65 \pm 2.27$, approximately $23.37 \pm 0.49$, approximately $52.35 \pm 3.34$, and approximately $67.70 \pm 2.44$, respectively.

These have been tested for unpredictability using the NIST randomness test. As for the NIST randomness test, the Applicant performed a series of statistical tests using National Institute of Standards and Technology Statistical Test Suite (NIST) in order to determine randomness quality of the random bits.

For reference, NIST tests are designed to quantitatively evaluate the randomness of binary sequences, and the NIST test suite consists of approximately 15 individual tests, each of which is designed to quantitatively measure various aspects of the randomness of the binary sequences.

The tests include frequency, block frequency, execution, longest running (LRO), continuation, approximate entropy, and cumulative sums (Cusums) evaluations, and the evaluation performed by the Applicant involved aggregating binary sequences from approximately 56 different random bits to ensure adequate stream length for approximately 7 statistical tests.

Here, P(s) is a path length distribution of a sample, s is a path length, $\tau$ is a delay time, I* is a transmission mean free path, and $\tau_o$ is a characteristic decay time of a medium.

Autocorrelation will be a value between approximately 0 and approximately 1, and as the time lag increases, the corresponding value should fall closer to approximately 0, which means that there is no longer any correlation compared to the first image. In order to check bio-inspired true random beat generation, the correlation time between two consecutive images in a time series of speckled pattern images was measured at approximately 50% point of as blood passed through the movement channel 430.

[Equation 1]

$$g_1(\tau) = \int_0^\infty P(s) exp\left[\left(-\frac{2\tau}{\tau_o}\right)\frac{s}{l^*}\right]$$

In addition, in order to evaluate data analysis (bit uniformity, average correlation) and the performance of random bits, digitized keys are examined. Here, the bit uniformity is evaluated by how balanced the distribution (ratio) of '0' and '1' bits is within the random bits.

[Equation 2]

$$Bit\ uniformity = \frac{1}{s}\sum_{l=1}^{s}K_l$$

The uniformity of the random bit sequence is often evaluated by determining the Hamming weight, which is the number of '1' bits in a binary string of length 's'. For a particular set of bits, the desired uniformity is achieved when the Hamming weight approaches approximately 0.5, reflecting an even distribution of '0' and '1' bits.

In addition, the correlation between images is investigated using a correlation matrix. In order to ensure objectivity of comparison, the random bits obtained through the method of the present invention were quantified and matched to create a uniform bit matrix to be examined (e.g., original speckle image: approximately 7.8 Mbits, CVN image: approximately 1.02 Mbits, CVN with SCI: approximately 1.8 Mbits, 2P-TO-VN image: approximately 3.3 Mbits, 2P-TO-VN with SCI: approximately 4.8 Mbits).

[Equation 3]

$$r = \frac{\sum_{i=1}^{n}(x_i - \bar{x})(y_i - \bar{y})}{\sqrt{\sum_{i=1}^{n}(x_i - \bar{x})^2}\sqrt{\sum_{i=1}^{n}(y_i - \bar{y})^2}}$$

Here, the Pearson correlation coefficient formula may be used to quantify the correlation. In Equation 3, $\bar{x}$ and $\bar{y}$ represent averages of the x values and y values, respectively, and the Pearson correlation coefficient, which is denoted by r, is closer to 0, indicating that there is no significant correlation between the two sets of random bits.

FIG. 13 is a table illustrating the results of the NIST statistical randomness test for the random numbers generated using the system according to an embodiment of the present invention, and the present Applicant can confirm that all tests passed.

The (a) of FIG. 12 is an exemplary image illustrating the original speckle image (OSI), the first processed image (CVN method), and the second processed image (2P-TO-VN method), and (b) of FIG. 12 illustrates an exemplary image to which image scrambling has been applied.

Referring to FIG. 12, the speckle pattern generated unpredictable random bits consisting of 0 and 1 bits, and additional randomness was incorporated using the image scrambling and the 2P-TO-VN method in order to improve high output and performance.

Referring to (b), the total pixels of the images containing the SCI are approximately 49980 (60x833) pixels (see (a)) and approximately 56520 (60x942) pixels (see (b)), respectively, and thus it can be seen that they are affected by scrambling.

According to an embodiment of the present invention, an innovative system for generating speckle patterns using a blood flow is presented.

In particular, the unique characteristics of blood, which is rich in various cellular components, can increase the randomness of the generated random numbers by improving the complexity and entropy of the generated speckle patterns.

The present invention can capture speckle patterns at the high speed of the system and requires only a very small amount of blood, which can improve system accessibility by minimizing both the cost and acquisition time associated with random number generation.

In addition, the system according to an embodiment of the present invention can increase the number of generated bits while providing overall unpredictability and strong security of the system, by applying the two-pass tuple-output von Neumann (2P-TO-VN) method, which is designed to prevent the generated random bits from being biased.

In addition, various other additional effects can be achieved by various embodiments of the present invention. These various effects of the present invention will be described in detail in each embodiment, or descriptions of effects that can be easily understood by those skilled in the art will be omitted.

As above, although the present invention has been described with limited embodiments and drawings, the present invention is not limited thereto, and various modifications and variations may be made by those skilled in the art in the technical field to which the present invention belongs within the technical idea of the present invention and the scope of equivalency of the claims described below.

**Claims**

1. A system for generating random numbers based on blood samples, comprising:

   a sample loading module configured to supply a preset blood sample;
   a light irradiation module configured to irradiate the blood sample provided by the sample loading module with light, the blood sample moved to a pre-designated region of interest (ROI) being irradiated with light;
   an image generation module configured to acquire an original speckle image by capturing an image of the blood sample located in the region of interest in a preset method using the light irradiation module; and
   a random number generation module configured to generate random numbers by processing the original speckle image generated from the image generation module in a preset method.

2. The system of claim 1, wherein
   the random number generation module includes
   a binary image generation unit configured to generate a binary image by performing a binarization process of classifying pixel values constituting the original speckle image into bit of 0 or 1.

3. The system of claim 2, wherein
   the random number generation module further includes
   a von Neumann extraction unit configured to generate a first processed image by applying a von Neumann post-processing method to the binary image acquired through the binary image generation unit.

4. The system of claim 3, wherein
   the von Neumann extraction unit is configured to
   generate a second processed image by further applying a two-pass tuple-output von Neumann (2P-TO-VN) method to the first processed image.

5. The system of claim 4, wherein
   the von Neumann extraction unit is configured to

   apply a one-pass tuple-output von Neumann (1P-TO-VN) method and
   process two bits as one set in a preset method, and the preset method is a method of removing one set if the one set is a duplication of the same bits.

6. The system of claim 5, wherein
   the von Neumann extraction unit is configured to
   , when a set is composed of different bits based on the remaining bits in a state where one set is the duplication of the same bits and the set is removed, maintain only a first bit or second bit constituting the set among the first bit and second bit.

7. The system of claim 6, wherein
   the von Neumann extraction unit is configured to
   regroup the removed set into a quad through the 1P-TO-VN method, and the quad is composed of a first set and a second set, and determine whether to maintain the removal of the first and second sets based on whether the first and second sets are the same.

8. The system of claim 7, wherein
   the von Neumann extraction unit is configured to
   restore the first and second sets when the first and second sets are different from each other.

9. The system of claim 4, wherein

   the random number generation module is configured to
   further include an image scrambling unit configured to generate a pre-processed image by performing image scrambling on the binary image generated through the binary image generation unit, and
   the von Neumann extraction unit is configured to
   generate the first and second processed images for the pre-processed image generated by the image scrambling unit.

10. A method of generating random numbers based on blood samples, comprising:

   (a1) supplying a blood sample through a sample loading module;
   (a2) irradiating the blood sample provided by the sample loading module with light through a light irradiation module, the blood sample moved to a pre-designated region of interest (ROI) being irradiated with light;
   (a3) acquiring an original speckle image by capturing an image of the blood sample located in the region of interest in a preset method using the light irradiation module; and
   (a4) generating random numbers by processing the original speckle image generated in the (a3) in a preset method.

11. The method of claim 10, wherein
   the (a4) includes

   (a41) generating a binary image by performing a binarization process of classifying pixel values constituting the original speckle image into bit of 0 or 1,
   (a42) generating a first processed image by applying a von Neumann post-processing method to the binary image acquired in the (a41), and
   (a43) generating a second processed image by further applying a 2P-TO-VN scheme to the first processed image generated in the (a42).

12. The method of claim 11, further comprising:
   before the (a42), (a42-0) generating a pre-processed image by performing image scrambling on the binary image generated in the (a41).

Fig. 1:

**10**

SAMPLE LOADING MODULE — 400

LIGHT IRRADIATION MODULE — 100

IMAGE GENERATION MODULE — 200

RANDOM NUMBER GENERATION MODULE — 300

BINARY IMAGE GENERATION UNIT — 310

Von Neumann EXTRACTION UNIT — 320

IMAGE SCRAMBLING UNIT — 330

Fig 2:

Fig 3:

ROI region (0.8 mm X 2 mm)

128 pixels

512 pixels

Fig 4:

512 pixels

128 pixels

Digitized key $K_n$      fps => 1250

$K_1$ = 1010100101010110101001.... (5.5M bits)

$K_2$ = 00101110100101001010010.... (5.5M bits)

$K_n$ = 1110010010100100100101.... (5.5M bits)

Fig 5:

ORIGINAL SPECKLE IMAGE

310

BINARIZATION PROCESS TO CLASSIFY INTO bit OF 0 OR 1

BINARY IMAGE GENERATION UNIT → BINARY IMAGE

330

IMAGE SCRAMBLING UNIT — SCRAMBLING → PRE-PROCESSED IMAGE

320

Von Neumann EXTRACTION UNIT — 1P-TO-VN → FIRST PROCESSED IMAGE

2P-TO-VN

SECOND PROCESSED IMAGE

Fig. 6:

Response $X$: 1 0 0 0 1 0 0 0 1 0 0 1 1 0 1 1 0 0 0 0 1 1 1

*1st Pass von Neumann extraction:*

2nd Pass von Neumann extraction:

Deblased Response $Y$:

Fig 7:

```
                    ┌─────────────┐
                    │    START    │
                    └──────┬──────┘
                           │
                           ▼
  ┌────────────────────────────────────────────────────────┐      S10
  │   STEP OF SUPPLYING BLOOD SAMPLE THROUGH SAMPLE         │
  │                 LOADING MODULE                          │
  └────────────────────────┬───────────────────────────────┘
                           │
                           ▼
  ┌────────────────────────────────────────────────────────┐      S20
  │  STEP OF IRRADIATING BLOOD SAMPLE PROVIDED BY SAMPLE    │
  │  LOADING MODULE WITH LIGHT THROUGH LIGHT IRRADIATION    │
  │  MODULE, AND IRRADIATING BLOOD SAMPLE MOVED TO PRE-     │
  │     DESIGNATED REGION OF INTEREST WITH LIGHT            │
  └────────────────────────┬───────────────────────────────┘
                           │
                           ▼
  ┌────────────────────────────────────────────────────────┐      S30
  │  STEP OF ACQUIRING ORIGINAL SPECKLE IMAGE BY CAPTURING  │
  │  IMAGE OF BLOOD SAMPLE LOCATED IN REGION OF INTEREST    │
  │   IN PRESET METHOD, USING LIGHT IRRADIATION MODULE      │
  └────────────────────────┬───────────────────────────────┘
                           │
                           ▼
  ┌────────────────────────────────────────────────────────┐      S40
  │       STEP OF GENERATING RANDOM NUMBERS BY             │
  │  PROCESSING ORIGINAL SPECKLE IMAGE GENERATED IN        │
  │          STEP S30 IN PRESET METHOD                    │
  └────────────────────────┬───────────────────────────────┘
                           │
                           ▼
                    ┌─────────────┐
                    │     END     │
                    └─────────────┘
```

Fig 8:

START

STEP OF GENERATING BINARY IMAGE BY PERFORMING BINARIZATION PROCESS OF CLASSIFYING PIXEL VALUES CONSTITUTING ORIGINAL SPECKLE IMAGE INTO BIT OF 0 OR 1 — S41

STEP OF GENERATING PRE-PROCESSED IMAGE BY PERFORMING IMAGE SCRAMBLING ON BINARY IMAGE GENERATED IN STEP S41 — S411

STEP OF GENERATING FIRST PROCESSED IMAGE BY APPLYING Von Neumann POST-PROCESSING METHOD TO BINARY IMAGE ACQUIRED IN STEP S41 — S42

STEP OF GENERATING SECOND PROCESSED IMAGE BY FURTHER APPLYING 2P-TO-VN METHOD TO FIRST PROCESSED IMAGE GENERATED IN STEP S42. — S43

END

Fig 9:

Fig. 10:

Fig. 11:

Fig.12:

(a)

(b)

Fig. 13

(a)

| NIST statistical test[1] | p-value | Proportion | Result[2] |
|---|---|---|---|
| Frequency | 0.000000 | 60/60 | Pass |
| BlockFrequency | 0.049821 | 60/60 | Pass |
| CumulativeSums | 0.000000 | 60/60 | Pass |
| | 0.000000 | 60/60 | Pass |
| Runs | 0.498475 | 59/60 | Pass |
| LongestRun | 0.505842 | 59/60 | Pass |
| ApproximateEntropy | 0.341105 | 58/60 | Pass |
| Serial | 0.466500 | 59/60 | Pass |
| | 0.449781 | 59/60 | Pass |

(b)

| NIST statistical test[1] | p-value | Proportion | Result[2] |
|---|---|---|---|
| Frequency | 0.000000 | 60/60 | Pass |
| BlockFrequency | 0.000000 | 60/60 | Pass |
| CumulativeSums | 0.000000 | 60/60 | Pass |
| | 0.000000 | 60/60 | Pass |
| Runs | 0.463253 | 60/60 | Pass |
| LongestRun | 0.462783 | 59/60 | Pass |
| ApproximateEntropy | 0.442474 | 59/60 | Pass |
| Serial | 0.439529 | 59/60 | Pass |
| | 0.497110 | 59/60 | Pass |

(1) The NIST statistical tests involve using a sequence of 4.8M bits obtained from 56 different random bits. This bit sequence is then divided into 60 sequences, each consisting of 80000 bits. The goodness-of-fit of the p-value distribution is compared to an expected distribution using the $\chi 2$ distribution. The bit sequence is considered to be random only if the resulting p-value is greater than or equal to 0.0001.
(2) A test is considered to have passed if the pass rate exceeds the minimum rate of greater than 56 out of 60 tests.

Europäisches
Patentamt

European
Patent Office

Office européen
des brevets

# EUROPEAN SEARCH REPORT

**Application Number**

EP 24 18 3738

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YOON INKWON ET AL: "Random Number Generators Utilizing Speckle Patterns from Blood Flow in Microfluidic Device", FRONTIERS IN OPTICS + LASER SCIENCE 2023, [Online] 9 October 2023 (2023-10-09), XP093228913, ISBN: 978-1-957171-29-6 Retrieved from the Internet: URL:https://opg.optica.org/abstract.cfm?URI=FiO-2023-JM4A.29> [retrieved on 2024-11-28] * figure 1 * * table 1 * * Title * * Sections 1 and 2 * | 1-12 | INV. G06F7/58 |
| X,P | YOON INKWON ET AL: "Blood-inspired random bit generation using microfluidics system", SCIENTIFIC REPORTS, [Online] vol. 14, no. 1, 29 March 2024 (2024-03-29) , XP093229278, US ISSN: 2045-2322, DOI: 10.1038/s41598-024-58088-6 Retrieved from the Internet: URL:https://www.nature.com/articles/s41598-024-58088-6.pdf> [retrieved on 2024-11-29] * the whole document * | 1-12 | |

**TECHNICAL FIELDS SEARCHED (IPC)**

G06F

-/--

The present search report has been drawn up for all claims

| Place of search | Date of completion of the search | Examiner |
|---|---|---|
| Munich | 29 November 2024 | Winkelbauer, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
......................................................................
& : member of the same patent family, corresponding document

EPO FORM 1503 03.82 (P04C01)

page 1 of 2

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROEL MAES ET AL: "Secure Key Generation from Biased PUFs", IACR, INTERNATIONAL ASSOCIATION FOR CRYPTOLOGIC RESEARCH, vol. 20150621:080549, 12 June 2015 (2015-06-12), pages 1-26, XP061018720, [retrieved on 2015-06-12] * Section 3 * | 1-12 | |

TECHNICAL FIELDS SEARCHED (IPC)

The present search report has been drawn up for all claims

| Place of search | Date of completion of the search | Examiner |
|---|---|---|
| Munich | 29 November 2024 | Winkelbauer, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
......................................................................................
& : member of the same patent family, corresponding document

EPO FORM 1503 03.82 (P04C01)

page 2 of 2

**EP 4 614 309 A1**

**Patent documents cited in the description**

- KR 101925787